## Europäisches Patentamt

## European Patent Office

(11) Publication number: **0 056 148**

## Office européen des brevets

**B1**

(12) **EUROPEAN PATENT SPECIFICATION**

(45) Date of publication of patent specification: **26.06.85**

(51) Int. Cl.⁴: **A 61 M 16/00**

(21) Application number: **81110774.7**

(22) Date of filing: **24.12.81**

(54) **Respirator.**

(30) Priority: **29.12.80 NL 8007074**

(43) Date of publication of application:
**21.07.82 Bulletin 82/29**

(45) Publication of the grant of the patent:
**26.06.85 Bulletin 85/26**

(84) Designated Contracting States:
**CH DE FR GB IT LI NL SE**

(56) References cited:
**FR-A-2 407 020**
**GB-A-2 026 326**
**GB-A-2 026 729**
**US-A-4 033 343**
**US-E- 29 383**

**ADVANCES IN INSTRUMENTATION, vol. 33,
part 1, 15-19 October 1978 PITTSBURGH (US)
A. LANGILL JR: "All Digital Control and
Metering of Fuel Gases to a Steam Boiler",
pages 269-274**

(73) Proprietor: **HONEYWELL B.V.**
**Rijswijkstraat 175**
**NL-1062 EV Amsterdam (NL)**

(72) Inventor: **Holland, Johannes**
**Van der Duin van Maasdamstraat 4**
**NL-4191 GC Geldermalsen (NL)**
Inventor: **van Liempd, Christinus Antoinetta
Paulus**
**Zonnebloemhof 10**
**NL-5482 RS Schijndel (NL)**
Inventor: **van Nuland, Robertus Josephus
Antonius**
**Piet Heijnlaan 55**
**NL-5694 CB Breugel (NL)**

(74) Representative: **Rentzsch, Heinz et al**
**Honeywell Europe S.A. Holding KG Patent- und
Lizenzabteilung Kaiserleistrasse 55**
**D-6050 Offenbach am Main (DE)**

Courier Press, Leamington Spa, England.

## Description

The invention relates to a respirator according to the general portion of Claim 1 as it is shown in GB—A 20 26 326. Furthermore, GB—A 20 26 729 describes an apparatus for controlled metering and mixing of two or more gases which also includes a control member with a narrow gas passage connected in series with at least one valve which can be alternately switched between a fully open position and a closed position by means of a control signal generated by a control element which is arranged to vary the relative amount of time during which the valve is open dependent on the desired flow rate. This apparatus may be used for artificial respiration.

It is the main object of the invention to provide a respirator in which the gas flow is very simply controlled and the gas stream to the patient is free of heavy fluctuations. For a respirator it is further essential that it works fail-safe. These objects are achieved by the invention as characterized in Claim 1. Choosing the inlet pressure and the cross-section of the gas passage in such a way that at any time when the valve is open the flow streams under critical conditions allows to measure only the inlet pressure and therewith facilitates control of the valves. Providing at least two valves connected in parallel and providing a phase-shift between the opening intervals of the two valves leads to an improved uniformity of the gas flow and simultaneously improves the reliability of the respirator as far as should one of the valves fail, gas will still be supplied to the patient via the other valves.

A critical passage is to be understood to mean herein a constriction in a duct whose cross-section is such that the gas velocity in the constriction equals the velocity of sound. It will be apparent that the gas velocity will depend on the pressure in the gas supply duct and on the cross-section of the constriction, and that the velocity of sound will depend on the properties of the gas and on the temperature.

The invention leads to a reliable respirator which can be finely adjusted and which has a large control range between minimum and maximum supply of more than 1:100. Further improvements and modifications of the invention are described in the Sub-claims.

The invention will be described in detail hereinafter with reference to the accompanying drawing. Therein:

Figure 1 is a longitudinal sectional view of a tube including a constriction.

Figure 2 is a graph illustrating the operation of the tube shown in Figure 1,

Figure 3 shows the flow characteristics of a number of critical passages,

Figure 4 shows a diagram of a control member for controlling a gas flow, including a critical passage, and

Figure 5 shows a diagram of an embodiment of a respirator in accordance with the invention.

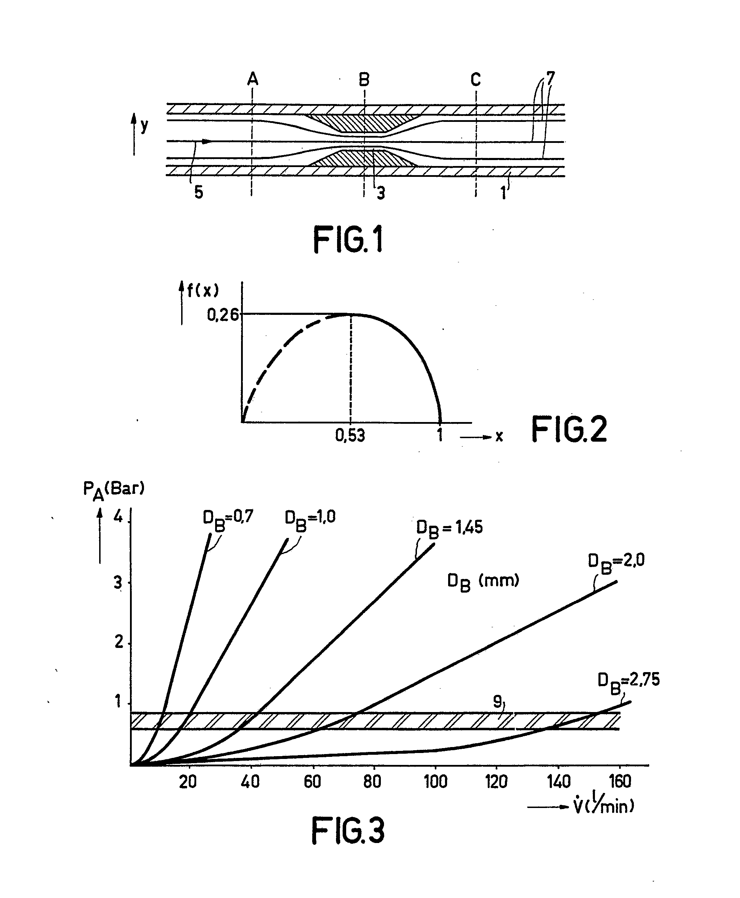

Some theoretical aspects of a gas flow through a tube including a constriction will first be described with reference to Figure 1. A tube 1 which is shown in longitudinal section in Figure 1, includes a constriction 3. Gas flows through the tube 1 in the direction of the arrow 5. Three stream lines 7 are shown in the figure.

In accordance with Euler's equation, the following is valid for a steady flow along a stream line in a frictionless medium:

$$\frac{1}{\rho} \frac{\delta p}{\delta s} + g \frac{\delta y}{\delta s} + \frac{1}{2} \frac{\delta v^2}{\delta s} = 0 \qquad (1)$$

Therein:

$\rho$ is the density of the gas

$p$ is the pressure of the gas

$s$ is the directional vector of the stream line

$y$ is the vertical coordinate (level)

$g$ the acceleration of the force of gravity

$v$ is the velocity of the gas.

If it is assumed that the gas does not exchange energy with its environment (isentropic process), furthermore:

$$\frac{P}{\rho k} = \text{constant} = C_1 \qquad (2)$$

Therein:

$$K = \frac{Cp}{Cv}$$

$C_p$ = specific heat at constant pressure

$C_v$ = specific heat at constant volume.

If it is also assumed that energy variation due to a change in level, is negligibly small and that the cross-section $F_B$ in the constriction 3 (at B in Figure 1) is much smaller than the cross-section $F_A$ upstream from the constriction (at A), so that $v_A \ll v_B$, it follows from (1) that:

$$v_B = \sqrt{\frac{2k}{K-1} \left( \frac{P_A}{\rho_A} - \frac{P_B}{\rho_B} \right)} \qquad (3)$$

For the mass flow $\phi_m$:

$$\phi_m = \rho_B \cdot F_B \cdot v_B = \rho_B F_B \sqrt{\frac{2K}{K-1} \left( \frac{P_A}{\rho_A} - \frac{P_B}{\rho_B} \right)} \qquad (4)$$

It follows from (2) that:

$$\frac{P_B}{\rho_B} = \frac{P_A}{\rho_A} \cdot x^{K-1/K} \qquad (5)$$

Therein,

$$x = \frac{P_B}{P_A}$$

and $0 < x < 1$.

If it is also assumed that the gas is an ideal gas, which holds good to a close approximation for those gases such as air, oxygen and laughing gas most often used in a respirator, the following is applicable:

$$P = \rho RT \tag{6}$$

Therein,

R is the gas constant, and

T is the absolute temperature of the gas.

It follows from (4), (5) and (6) that:

$$\phi_m = F_B P_A \sqrt{\frac{2K}{K-1} \cdot \frac{1}{RT} \, (x^{2/K} - x^{K+1/K})} \tag{7}$$

The term

$$\sqrt{\frac{2K}{K-1} \cdot \frac{1}{RT}}$$

is constant for a given gas at a given temperature and is approximately constant as the ambient temperature varies, because T is the absolute temperature and occurs after the radical sign. In practice, this term changes approximately 1.7 ‰ per °C change in ambient temperature.

The function

$$f(x) = \sqrt{x^{2/K} - x^{K+1/K}}$$

is graphically represented in Figure 2 for $0 < x < 1$ and K=1.40. This value of K is applicable to air.

The maximum of f(x) is at:

$$x_m = \left(\frac{2}{K+1}\right)^{K/K-1} = \frac{P_m}{P_A} = 0.53$$

The pressure $P_c$ at C is approximately equal to the ambient pressure. When $P_A > P_c$, gas starts to flow; then, in accordance with (7):

$$\phi_m = F_B \cdot \sqrt{\frac{2K}{K-1} \cdot \frac{1}{RT}} \cdot P_A \cdot f(x) \tag{8}$$

This formula is correct if $P_A < P_m$.

If $P_A = P_m$, it holds good for $P_m \approx P_c = 1$ bar that the critical value of p, ρ and v are equal to:

$$P_{Akr} = \frac{1}{0.53} = 1.9 \text{ bar}$$

$$v_{Bkr} = \sqrt{KRT_B} = C \text{ (velocity of sound)}$$

$$\rho_{Bkr} = \left(\frac{2}{K+1}\right)^{1/K-1} \cdot \frac{P_A}{RT}$$

When the pressure $P_A$ is increased further, f(x) does not decrease but remains constant at the maximum value. In the narrowest cross-section 3, the gas velocity then remains equal to the velocity of sound. This is because a higher value is impossible. A constriction of this kind is referred to as a critical passage. The mass flow in the narrowest cross-section 3 is determined by the density of the gas $\rho_B$ and is proportional to the pressure $P_A$.

In Figure 3, the volume flow $\dot{v}$ measured for a number of critical passages is shown as a function of the pressure $P_A$. The diameter of the constriction $D_B$ is stated in millimeters for each curve. This figure clearly shows that beyond a shaded pressure zone 9 the volume flow varies linearly with the supply pressure $P_A$. Assuming that the temperature of the outflowing gas ultimately reaches a constant value of 293 K and the pressure a constant value of 1 bar, this linear relationship also applies to the mass flow, of course.

It will be apparent from the foregoing that for a critical passage a linear relationship exists between the pressure of the gas supplied and the mass flow through the critical passage. This relationship is determined completely by measurable physical variables, such as the area and the geometry of the narrowest cross-section and the properties of the gas.

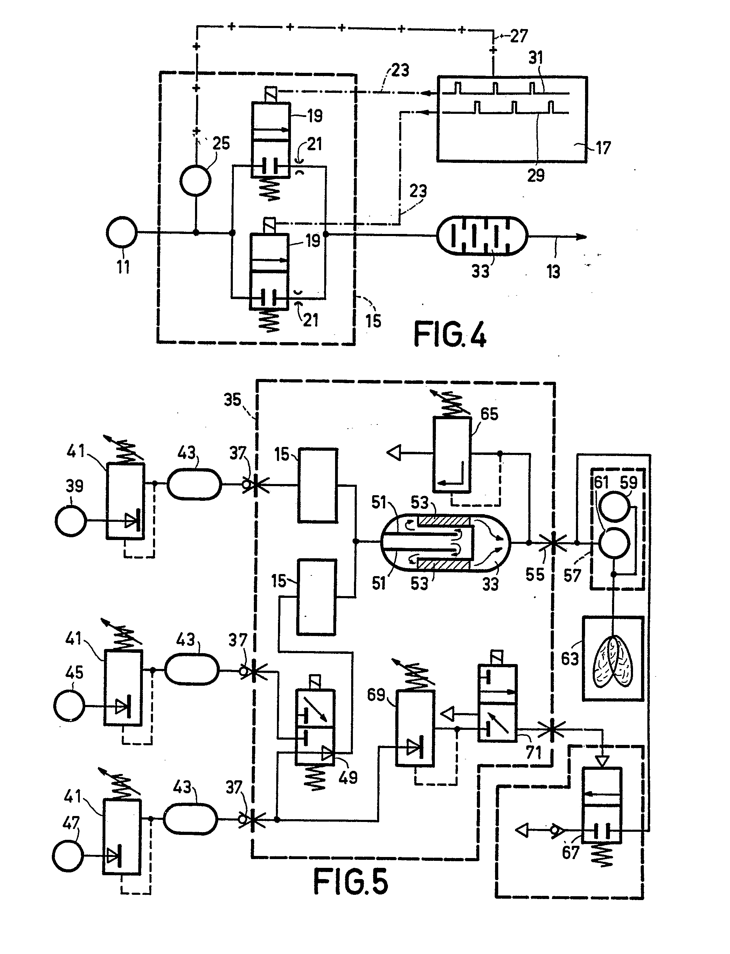

Figure 4 shows a diagram of a control member for controlling a gas flow in a gas duct which extends from a gas supply duct 11 to a patient duct 13. The control member comprises a gas flow controller 15 and a control element 17. The gas flow controller 15 comprises two parallel connected valves 19, each of which is connected in series with a critical passage 21. The critical passages 21 may be formed, for example, by intermediate duct portions including a constriction as shown in Figure 1, or by the narrow exit openings of the valves 19. The diameter in the region of the constriction preferably amounts to approximately 1.5 mm.

The valves 19 are opened and closed by control signals which are applied by the control element 17 *via* control leads 23. When a valve is open, the gas flow through the valve is linearly dependent on the pressure in the supply duct 11, as has already been demonstrated. This pressure is measured by means of a pressure gauge 25 and is applied, *via* a measuring connection 27, to the control element 17. The desired gas flow can be adjusted, for example, by means of an adjusting knob (not shown) provided on the control element 17. The control element then varies the relative amount of time during which the valves 19 are open in dependence on the pressure in the gas supply duct 11 and the adjusted, desired gas flow. In principle, one valve 19 with the associated critical passage 21 would suffice for this control system, but the parallel connection of a plurality of valves offers the advantage that the control range may be larger and the conducted gas flow is more uniform (less pulsatory). The latter can be achieved by controlling the valves with a mutual difference in phase; in the embodiment shown in Figure 4, this is realized, for example, so that the period during which one valve is open is situated in the middle of the period during which the other valve is closed. For the purposes of illustration, Figure 4 shows examples of control signals 29 and 31 for the two valves 19. The relevant valve is open during each positive pulse.

The repetition frequency of the two pulse series is, for example, 15 Hz.

A further step for suppressing irregularities in the gas flow is the introduction of a buffer between the critical passage 21 and the patient duct 13, for example, a buffer in the form of an acoustic filter 33 which also serves to suppress disturbing noise.

Figure 5 shows a diagram of a respirator utilizing control members of the kind described with reference to Figure 4. For the sake of the simplicity, Figure 5 shows only those parts through which the gases pass, the control elements 17 and other known electrical components having been omitted.

The respirator shown in Figure 5 comprises a housing 35 in which there are accommodated two gas flow controllers 15 which in principle correspond to the gas flow controller shown in Figure 4, but each of which comprises four parallel connected valves 1a instead of two, so that the controllability and uniformity of the gas flow are even further improved.

One of the gas flow controllers 15 (the upper controller in Figure 5) is connected to an oxygen duct 39 via a gas coupling 37. Between the oxygen duct 39 and the gas coupling 37 there are provided a reducing valve 41 and a buffer 43. The reducing valve 41 ensures that the oxygen is applied to the gas flow controller at an adjustable pressure (for example, 3—6 bar) and the buffer 43 smooths out any pulses in the gas flow produced by the gas flow controller, so that these pulses do not have a disturbing effect on the reducing valve.

A laughing gas duct 45 and an air duct 47 are similarly connected to gas couplings 37 via reducing valves 41 and buffers 43. The latter gas couplings are connected to the second gas flow controller 15 via a change-over valve 49. The change-over valve 49 is controlled so that air or laughing gas is applied to the second gas flow controller 15, as desired. The first gas flow controller 15 thus controls the oxygen flow and the second gas flow controller controls the flow of air or $N_2O$. The ratio between on the one hand the amount of oxygen and on the other hand the amount of air or $N_2O$ can be controlled by suitable control of the two gas flow controllers 15.

The gas flow emerging from the gas flow controllers 15 is passed through an acoustic filter 33 which comprises guide partitions 51 which force the gas flow (denoted by arrows) through fibrous material 53. The gas flows from the two gas flow controllers 15 are also thoroughly mixed in this filter.

Via a gas coupling 55, the outlet of the filter 33 is connected to a nozzle 57 which may include a pressure detector 59 and a gas flow detector 61.

Via this nozzle, the gas flow is administered to a patient 63.

The outlet of the filter 33 is connected to a safety valve 65 which is automatically opened when the patient pressure rises above a preset value, (for example, from 0.025 to 0.12 bar above atmospheric pressure).

For exhalation there is provided an exhalation valve 67 which can be disposed of after use. This exhalation valve is pneumatically controlled by means of air from the air duct 47 which is supplied via a reducing valve 69 and a valve 71.

## Claims

1. A respirator for controlling a gas flow to a patient at a predetermined dose rate, comprising at least one control member for controlling a gas flow in a gas duct which extends from the gas supply duct (11) to a patient duct (13), said control member (15) comprising a narrow gas passage (21) connected in series with at least one valve (19) which can be alternately switched between a fully open position and a closed position by means of a control signal (29, 31) generated by a control element (17) which is arranged to vary the relative amount of time during which the valve is open in dependence on the desired flow rate and whereat the gas passage (21) in the open position of the valve (19) forms a critical flow passage; characterized in that

at least two valves (19), each followed by a critical passage (21), are connected in parallel and provided in the gas path;

the control element (17) is arranged to control the valves with a mutual difference in phase and

a buffer (33) is provided between the critical passages (21) and the patient duct (13) for suppressing irregularities in the gas flow to the patient.

2. A respirator according to Claim 1 characterized in that between the gas supply duct (11) and the critical passage (21) there is provided a pressure detector (25) which is also connected to the control element (17) so as to vary the relative amount of time during which the valves (19) are open in dependence on the pressure measured by the pressure detector.

3. A respirator according to Claim 1 or 2 characterized in that at least four valves (19) are connected in parallel.

## Patentansprüche

1. Beatmungsgerät zur dosierten Steuerung des Gasstroms zu einem Patienten, mit wenigstens einem Stellglied zur Steuerung eines Gasstroms durch eine sich von einer Gasversorgungsleitung (11) zu einer Patienten-Anschlußleitung (13) erstreckenden Gasleitung, wobei

das Stellglied (15) einen engen Gasdurchlaß (21) aufweist, der mit wenigstens einem abwechselnd zwischen einer Schließstellung und einer voll geöffneten Stellung umschaltbaren Ventil (19) in Reihe geschaltet ist,

die Umschaltung des Ventils (19) durch ein von einem Steuergerät (17) erzeugtes Steuersignal (29, 31) erfolgt, das Steuergerät (17) dabei die relative Öffnungsdauer des Ventils in Abhängigkeit von der gewünschten Dosierung ändert,

und der Gasdurchlaß (21) bei geöffnetem Ventil (19) eine Engstelle mit kritischer Strömungsgeschwindigkeit bildet; dadurch gekennzeichnet, daß wenigstens zwei jeweils mit einer kritischen Engstelle (21) in Reihe geschaltete Ventile (19) parallel zueinander in den Gasweg eingeschaltet sind;

das Steuergerät (17) die Ventile gegeneinander phasenversetzt ansteuert

und zwischen die kritischen Engstellen (21) und die Patientenanschlußleitung (13) ein Pufferraum (33) eingeschaltet ist, um Unregelmäßigkeiten im Gasstrom zum Patienten zu unterdrücken.

2. Beatmungsgerät nach Anspruch 1, dadurch gekennzeichnet, daß zwischen der Gasversorgungsleitung (11) und der kritischen Engstelle (21) ein Druckfühler (25) angeordnet ist, der außerdem an das Steuergerät angeschlossen ist, um die relative Öffnungsdauer der Ventile in Abhängigkeit von dem durch den Druckfühler gemessenen Druck zu verändern.

3. Beatmungsgerät nach Anspruch 1 oder 2, dadurch gekennzeichnet, daß wenigstens vier Ventile (19) parallelgeschaltet sind.

**Revendications**

1. Respirateur pour contrôler un courant de gaz envoyé à un patient à un taux de dosage prédéterminé, comprenant au moins un organe de réglage pour régler un débit de gaz dans un conduit de gaz qui s'étend du conduit d'alimentation en gaz (11) jusqu'à un conduit de patient (13), l'organe de réglage (15) comprenant un passage de gaz étroit (21) connecté en série avec au moins une soupape (19) qui peut être alternativement changée entre une position complètement ouverte et une position fermée au moyen d'un signal de commande (29, 31) engendré par un élément de commande (17) qui est agencé pour faire varier la quantité relative de temps pendant laquelle la soupape est ouverte en fonction du débit de gaz voulu et où le passage de gaz (21) constitue un passage critique dans la position ouverte de la soupape (19); caractérisé en ce qu'au moins deux soupapes (19), dont chacune est suivie par un passage critique (21), sont connectées en parallèle et prévues dans le chemin de gaz; en ce que l'élément de commande (17) est agencé pour commander les soupapes avec un déphasage entre elles et en ce qu'un tampon (33) est prévu entre les passages critiques (21) et le conduit de patient (13) pour supprimer les irrégularités du courant de gaz jusqu'au patient.

2. Respirateur selon la revendication 1, caractérisé en ce qu'un détecteur de pression (25), également connecté à l'élément de commande (17), est prévu entre le conduit d'alimentation en gaz (11) et le passage critique (21), de manière à faire varier la quantité relative de temps pendant laquelle les soupapes (19) sont ouvertes en fonction de la pression mesurée par le détecteur de pression.

3. Respirateur selon l'une des revendications 1 ou 2, caractérisé en ce qu'au moins quatre soupapes (19) sont connectées en parallèle.

FIG.1

FIG.2

FIG.3

0 056 148

FIG.4

FIG.5

2